# EUROPEAN PATENT SPECIFICATION

(11) **EP 3 464 531 B1**
(45) Date of publication and mention of the grant of the patent: **19.02.2020**
(21) Application number: 17724711.1
(22) Date of filing: 10.05.2017
(51) Int. Cl.: C11D 1/12, C11D 1/37, C11D 3/20, C11D 10/04, C11D 17/00

(54) **CLEANSING COMPOSITION**
REINIGUNGSMITTELZUSAMMENSETZUNG
COMPOSITION DE NETTOYAGE

(30) Priority: 26.05.2016 US 201662341934 P
(43) Date of publication of application: 10.04.2019
(73) Proprietor: Innospec Active Chemicals LLC, High Point, NC 27260 (US)
(72) Inventor: COHEN, Mitchell J., High Point, North Carolina 27260 (US); COTRELL, Phillip L., High Point, North Carolina 27260 (US)
(74) Representative: Appleyard Lees IP LLP
(86) International application number: PCT/US2017/031869
(87) International publication number: WO 2017/205051

(56) References cited:
- EP-A2- 0 176 330
- WO-A1-94/09763
- WO-A1-2013/093473
- US-A- 5 866 144

## Description

### FIELD OF THE INVENTION

The present invention relates to cleansing compositions, particularly solid cleansing compositions. The present invention also relates to a base composition for use in a cleansing composition, to a method of reducing and/or eliminating grit formation in a cleansing composition, to a method of cleansing the skin and/or hair and to a personal care product comprising a cleansing composition.

### BACKGROUND OF THE INVENTION

Cleansing compositions in the form of a solid bar are well known. These can be in the form of soap bars (comprising mainly soap, for example in the form of fatty acid salts) or in the form of syndet bars, containing synthetic detergent(s) (or surfactant(s)) and little or no soap. Soap bars typically provide good cleansing and foaming and are relatively cheap to produce. but have a high pH and can cause irritation to the skin of a user. Syndet bars typically have a lower pH and are milder than soap bars, but are significantly more expensive to make. Therefore, combination or combo bars (hereinafter referred to as combo bars) were developed, which comprise a mixture of both soap and synthetic detergent(s).

An example of a combo bar is a beauty bar, which comprises high levels of synthetic detergent(s) and low levels of soap. Due to the high levels of synthetic detergent(s), beauty bars typically exhibit many of the problems associated with syndet bars and are expensive to produce. It therefore would be desirable to provide a combo bar having a higher level of soap, to provide good cleansing and keep the production costs low, in combination with a lower level of synthetic detergent(s), to lower the overall pH and reduce irritation in use. However, it is well known in the art that such combo bars are difficult to formulate and produce. This is because particulates known in the art as "grit" tend to form in the combo bars under running water at temperatures of about 20 to 40°C. It is believed that the grit forms because the components of the combo bars have different solubilities and melting temperatures. Such grit is undesirable and makes this type of combo bar unattractive to the user.

For example, it is considered desirable to combine soap with a synthetic detergent in the form of chips comprising acyl isethionate, free fatty acid and alkali metal isethionate. Typically such chips are made from the direct esterification of free fatty acids and alkali metal isethionates to form so-called "DEFI" (i.e. directly esterified fatty acid isethionate) chips. Adding soap to "DEFI" chips or flakes reduces cost (soap being much cheaper), while adding such chips or flakes to a predominantly soap composition reduces harshness of soaps. However, mixing such "DEFI" flakes or chips with soap in a conventional process results in bars with a high degree of "grittiness". Thus it has been traditionally true that combining too much soap with such chips or flakes, or adding too much chips or flakes to predominantly soap bars has resulted (when chips or flakes are co-extruded with soap chips) in bars having poor user properties. Without wishing to be bound by theory, this is believed to be caused by differences in wear rates between the chips or flakes comprising acyl isethionate and the soap chips, thereby resulting in the so-called "grittiness" problem.

Attempts have been made to overcome the problem discussed above of grit in combo bars. For example, combo bars have been prepared using a molten process (see, for example WO 2002/012430) but such a process requires specialised equipment and is expensive to conduct.

US 5,866,144 discloses a solid composition comprising 27 to 38 wt. % of sodium cocoylisethionate, 18 to 30 wt. % soap, 27 to 33 wt. % free fatty acid, 1 to 5 wt. % of paraffin wax, and the balance water, with the proviso that no other surfactant is present in the composition other than soap and sodium cocoylisethionate; said composition providing good lathering, conditioning, mildness, good hardness, slough and use-up values.

Therefore, there remains a need for a combo bar which contains a relatively high level of soap but which has reduced or no grit formation, it is an aim of the present invention to provide an improved cleansing composition, for example a solid cleansing composition such as a combo bar, particularly which comprises a high proportion of soap and exhibits reduced or eliminated grit formation and which can be made by conventional manufacturing processes.

### SUMMARY OF THE INVENTION

In a first aspect, the present invention provides a cleansing composition comprising:
(i) from 1 to 15 wt% of at least one C₈-C₂₂ fatty acid;
(ii) from 2 to 25 wt% of at least one isethionate ester surfactant of the formula (I): 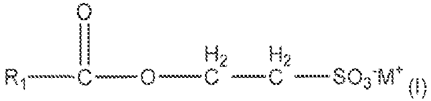
(iii) from 0.5 to 7.5 wt% of at least one alkyl isethionate ester surfactant of the formula (II): 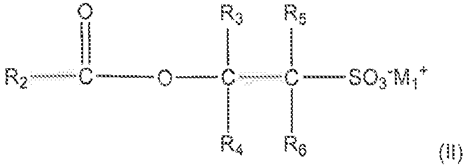
   wherein R₁ and R₂ each independently represents a substituted or unsubstituted C₄-C₃₆ hydrocarbyl group;
   R₃, R₄, R₅ and R₅ each independently represents hydrogen or a substituted or unsubstituted C₁-C₄ alkyl group, provided that at least one of R₃, R₄, R₅ and R₆ is not hydrogen; and
   M⁺ and M₁⁺ each independently represents a cation; and
(iv) from 52.5 to 95 wt% of soap.

In a second aspect, the present invention provides a method of cleansing the skin and/or hair comprising contacting the skin and/or hair with a cleansing composition according to the first aspect.

In a third aspect, the present invention provides a method of manufacturing a cleansing composition having reduced and/or eliminated grit formation, the method comprising admixing:
(i) from 1 to 15 wt% of at least one C₈-C₂₂ fatty acid;
(ii) from 2 to 25 wt% of at least one isethionate ester surfactant of the formula (I): 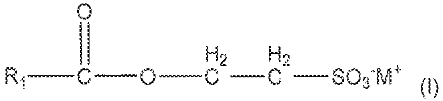
(iii) from 0.5 to 7.5 wt% of at least one alkyl isethionate ester surfactant of the formula (II): 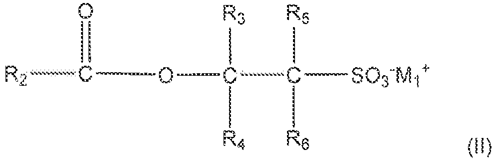
   wherein R₁ and R₂ each independently represents a substituted or unsubstituted C₄-C₃₅ hydrocarbyl group;
   R₃, R₄, R₅ and R₆ each independently represents hydrogen or a substituted or unsubstituted C₁-C₄ alkyl group, provided that at least one of R₃, R₄, R₅ and R₆ is not hydrogen; and
   M⁺ and M₁⁺ each independently represents a cation; and
(iv) from 52.5 to 95 wt% of soap.

The components (i), (ii) and (iii) of the cleansing composition of the first aspect of the present invention may be considered to represent a base composition, i.e. which is useful in the cleansing composition. In other words, the cleansing composition of the first aspect of the present invention may be considered to comprise a base composition and soap. wherein the base composition comprises the components (i), (ii) and (iii) defined herein.

In a fourth aspect, the present invention provides a base composition for use in a cleansing composition, the base composition comprising:
(i) from 10 to 40 wt% of at least one C₈-C₂₂ fatty acid; and
(ii) from 60 to 90 wt% of a synthetic detergent composition, wherein the synthetic detergent composition comprises from 65 to 95 wt% of at least one isethionate ester surfactant of the formula (I): 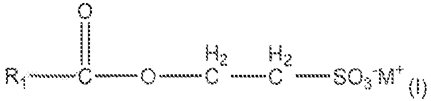 and from 5 to 35 wt% of at least one alkyl isethionate ester surfactant of the formula (II): 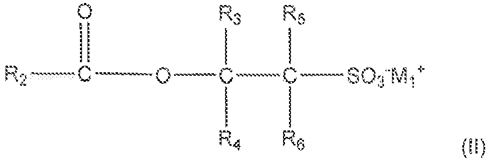
   wherein R₁ and R₂ each independently represents a substituted or unsubstituted C₄-C₃₆ hydrocarbyl group;
   R₃, R₄, R₅ and R₆ each independently represents hydrogen or a substituted or unsubstituted C₁-C₄ alkyl group, provided that at least one of R₃, R₄, R₅ and R₆ is not hydrogen; and
   M⁺ and Mi⁺ each independently represents a cation.

In a fifth aspect, the present invention provides a cleansing composition comprising a base composition according to the fourth aspect of the invention.

In a sixth aspect, the present invention provides a method of reducing and/or eliminating grit formation in a cleansing composition, the method comprising incorporating a base composition according to the fourth aspect of the invention into the cleansing composition.

In a seventh aspect, the present invention provides the use of a base composition according to the fourth aspect of the invention to reduce and/or eliminate grit formation in a cleansing composition.

In an eighth aspect, the present invention provides method of cleansing the skin and/or hair comprising contacting the skin and/or hair with a cleansing composition comprising a base composition according to the fourth aspect of the invention.

In a ninth aspect, the present invention provides a personal care product comprising a cleansing composition according to the first aspect of the invention and packaging.

### BRIEF DESCRIPTION OF DRAWINGS

For a better understanding of the invention, and to show how exemplary embodiments of the same may be carried into effect, reference will be made, by way of example only, to the accompanying diagrammatic Figures, in which:

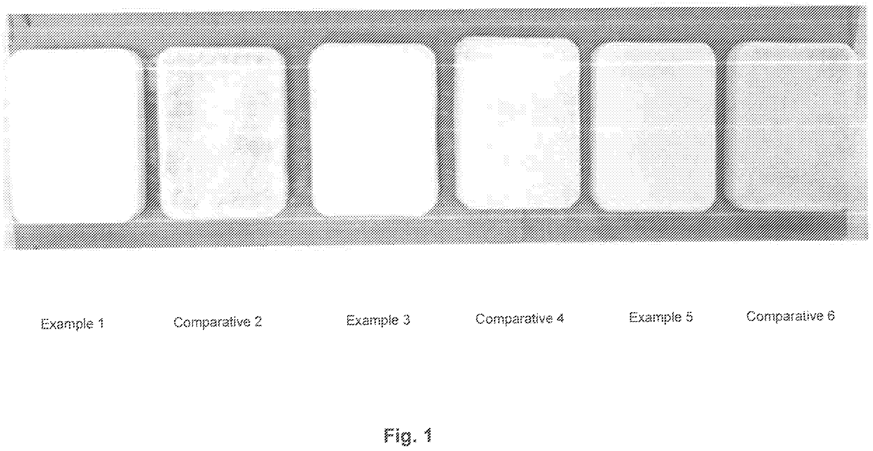

Figure 1 shows the amount of grit for bars according to the invention compared to the comparative examples.

### DETAILED DESCRIPTION OF THE INVENTION

Unless otherwise stated, the following terms used in the specification and claims have the meanings set out below.

The term "fatty acid" is used herein in its ordinary sense, which is well-known to those skilled in the art. Specifically, it refers to a nonesterified fatty acid.

As used herein, the term "hydrocarbyl group" is used in its ordinary sense, which is well-known to those skilled in the art. Specifically, it refers to a group having a carbon atom directly attached to the remainder of the molecule and having predominantly hydrocarbon character. Examples of hydrocarbyl groups include hydrocarbon groups, i.e. aliphatic (which may be saturated or unsaturated, linear or branched, for example alkyl or alkenyl), alicyclic (for example cycloalkyl, cycloalkenyl) and aromatic (for example phenyl) groups.

The term "alkyl" includes both straight and branched chain alkyl groups. References to individual alkyl groups such as "propyl" are specific for the straight chain version only and references to individual branched chain alkyl groups such as "isopropyl" are specific for the branched chain version only. For example, "C₄-C₃₆ alkyl" includes C₁₀-C₃₆, C₄-C₆ alkyl, propyl, isopropyl and t-butyl.

The term "alkenyl" includes both straight and branched chain alkenyl groups. References to individual alkenyl groups such as "propenyl" are specific for the straight chain version only and references to individual branched chain alkenyl groups such as "isopropenyl" are specific for the branched chain version only. For example, "C₄-C₃₆ alkenyl" includes C₁₀-C₃₆ alkenyl, C₄-C₆ alkenyl, propenyl and isopropenyl.

The term "alkoxy" includes both straight and branched chain alkoxy groups. References to individual alkoxy groups such as "propoxy" are specific for the straight chain version only and references to individual branched chain alkyl groups such as "isopropoxy" are specific for the branched chain version only. For example, "C₁-C₄ alkoxy" includes C₁-C₂ alkoxy, propoxy, isopropoxy and t-butoxy.

The term "aryl" means a cyclic or polycyclic aromatic ring having from 5 to 12 carbon atoms. Examples of aryl groups include, but are not limited to, phenyl, biphenyl and naphthyl. In a particular embodiment, an aryl group may be phenyl.

The term "C₄-C₃₁ alkyl-C₅-C₁₂ aryl" means a C₅-C₁₂ aryl group covalently attached to a C₄-C₃₁ aikyi group, both of which are defined herein.

References to "a solid composition" herein refer to compositions which are in the solid state under normal atmospheric conditions (i.e. at a pressure of 1 atmosphere and 298 K).

References to "soap" herein refer to compounds commonly known as soap, for example the alkali metal and alkanol ammonium salts of aliphatic alkane or alkene monocarboxylic acids.

Throughout this specification, the term "comprising" or "comprises" means including the component(s) specified but not to the exclusion of the presence of other components. The term "consisting essentially of or "consists essentially of means including the components specified but excluding other components except for components added for a purpose other than achieving the technical effect of the invention. The term "consisting of" or "consists of' means including the components specified but excluding other components.

Whenever appropriate, depending upon the context, the use of the term "comprises" or "comprising" may also be taken to include the meaning "consists essentially of" or "consisting essentially of", and also may also be taken to include the meaning "consists of" or "consisting of".

In this specification, unless otherwise indicated any amounts referred to relate to the amount of active component present in the composition. The skilled person will appreciate that commercial sources of some of the components referred to herein may include impurities, side-products and/or residual starting material. However the amounts specified refer only to the active material and do not include any impurity, side-product, starting material or diluent that may be present.

The optional features set out herein may be used either individually or in combination with each other where appropriate and particularly in the combinations as set out in the accompanying claims. The optional features for each exemplary embodiment of the invention, as set out herein are also applicable to any other aspects or exemplary embodiments of the invention, where appropriate. In other words, the skilled person reading this specification should consider the optional features for each aspect or embodiment of the invention as interchangeable and combinable between different aspects of the invention.

### Cleansing Composition

In a first aspect, the present invention provides a cleansing composition. The cleansing composition comprises:
(i) from 1 to 15 wt% of at least one C₈-C₂₂ fatty acid;
(ii) from 2 to 25 wt% of at least one isethionate ester surfactant of the formula (I): 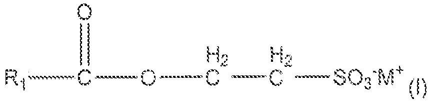
(iii) from 0.5 to 7.5 wt% of at least one alkyl isethionate ester surfactant of the formula (II): 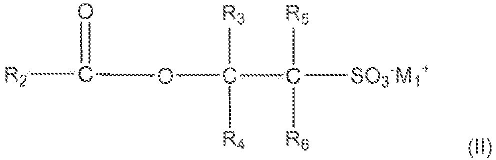
   wherein R₁ and R₂ each independently represents a substituted or unsubstituted C₄-C₃₆ hydrocarbyl group;
   R₃, R₄, R₅ and R₆ each independently represents hydrogen or a substituted or unsubstituted C₁-C₄ alkyl group, provided that at least one of R₃, R₄, R₅ and R₆ is not hydrogen; and
   M⁺ and M₁⁺ each independently represents a cation; and
(iv) from 52.5 to 95 wt% of soap.

Suitably, the cleansing composition of the first aspect is a solid composition. For example, the cleansing composition may be in the form of a solid bar or block. The cleansing composition of the first aspect may be a combo bar as discussed above, for example a combo bar having a relatively high proportion of soap. Suitably, the cleansing composition of the first aspect is a personal cleansing composition, for example which is suitable for cleansing the skin and/or hair.

The proportion of the isethionate ester surfactant of the formula (I) and alkyl isethionate ester surfactant of the formula (II) in the cleansing composition according to the first aspect is believed to provide a cleansing composition (especially a solid cleansing composition, such as a combo bar) which does not form or forms lower than previously achieved levels of grit when the composition is contacted with water at temperatures of about 20 to 40°C. This is advantageous in terms of the performance of the cleansing composition in use and the perception of the users. The ability to formulate the isethionate ester surfactant of the formula (I) and alkyl isethionate ester surfactant of the formula (II) with relatively high levels of soap further provides a cleansing composition (especially a solid cleansing composition) which provides good cleansing and foaming and which is mild to the skin and/or hair. Additionally, cleansing compositions according to the first aspect can be manufactured using standard equipment and are relatively cheap to produce.

Suitably, the cleansing composition of the first aspect comprises from 1 to 12 wt% of at least one C₈-C₂₂ fatty acid, such as from 5 to 10 wt% of at least one C₈-C₂₂ fatty acid, particularly from 8 to 9 wt% of at least one C₈-C₂₂ fatty acid.

Any suitable C₈-C₂₂ fatty acid may be included in the cleansing composition of the first aspect For example, the C₈-C₂₂ fatty acid may be selected from one or more C₈-C₂₂ saturated or unsaturated fatty acids, such as one or more of stearic acid, coconut fatty acid, lauric acid, myristic acid and palmitic acid.

In some embodiments only a single C₈-C₂₂ fatty acid may be present in the cleansing composition of the first aspect. In some embodiments a mixture of two or more C₈-C₂₂ fatty acids may be present. In such embodiments the above amounts refer to the total amounts of all C₈-C₂₂ fatty acids present in the composition.

The C₈-C₂₂ fatty acid is believed to provide advantages in relation to the processing, for example moulding, of the cleansing composition, for example into bars and/or blocks. The C₈-C₂₂ fatty acid is believed to reduce the melting point of the cleansing composition so as to make it more pliable for processing.

Suitably, the cleansing composition of the first aspect comprises from 5 to 20 wt% of at least one isethionate ester surfactant of the formula (I), such as from 5 to 15 wt% of at least one isethionate ester surfactant of the formula (I), particularly from 8 to 12 wt% of at least one isethionate ester surfactant of the formula (I).

In the formula (I), the group R₁ represents a substituted or unsubstituted C₄-C₃₆ hydrocarbyl group and M⁺ represents a cation.

Preferably, R₁ is selected from a substituted or unsubstituted C₄-C₃₆ alkyl, C₄-C₃₆ alkenyl, Cs-C₁₂ aryl or C₄-C₃₁ alkyl-C₅-C₁₂ aryl group. More preferably. R₁ is selected from a substituted or unsubstituted C₄-C₃₆ alkyl or C₄-C₃₆ alkenyl group. Most preferably, R₁ is an unsubstituted C₄-C₃₆ alkyl or C₄-C₃₆ alkenyl group, especially an unsubstituted C₄-C₃₆ alkyl group.

Preferably, R₁ represents a substituted or unsubstituted C₅-C₃₀ alkyl group, preferably a substituted or unsubstituted C₇-C₂₄ alkyl group, more preferably a substituted or unsubstituted C₇-C₂₁ alkyl group, most preferably a substituted or unsubstituted C₁-C₁₇ alkyl group.

Preferably, R₁ represents an unsubstituted C₅-C₃₀ alkyl group, preferably an unsubstituted C₇-C₂₄ alkyl group, more preferably an unsubstituted C₇-C₂₁ alkyl group, most preferably an unsubstituted C₇-C₁₇ alkyl group.

R₁ is preferably the residue of a fatty acid. Fatty acids obtained from natural oils often include mixtures of fatty acids. For example, the fatty acid obtained from coconut oil contains a mixture of fatty acids including C₁₂ lauric acid, C₁₄ myristic acid, C₁₆ palmitic acid. C₈ caprylic acid, and C₁₈ stearic and oleic acid.

R₁ may include the residue of one or more naturally occurring fatty acids and/or of one or more synthetic fatty acids. In some preferred embodiments, R₁ consists essentially of the residue of a single fatty acid.

Examples of carboxylic acids from which R₁ may be derived include coco acid, hexanoic acid, caproic acid, caprylic acid, capric acid, lauric acid, myristic acid, palmitic acid, palmitoleic acid, stearic acid, oleic acid, linoleic acid, arachidic acid, gadoleic acid, arachidonic acid, eicosapentanoic acid, behinic acid, eruic acid, docosahexanoic lignoceric acid, naturally occurring fatty acids such as those obtained from coconut oil, tallow, palm kernel oil, butterfat, palm oil, olive oil, corn oil, linseed oil, peanut oil, fish oil and rapeseed oil; synthetic fatty acids made as chains of a single length or a selected distribution of chain lengths; and mixtures thereof. Suitably R₁ comprises the residue of coco acid, the residue of mixed fatty acids derived from coconut oil or the residue of mixed fatty acids derived from palm kernel oil.

For example, the isethionate ester surfactants of the formula (I) may be selected from one or more of sodium lauroyl isethionate, sodium cocoyl isethionate and sodium myristoyl isethionate. Sodium cocoyl isethionate is especially preferred.

The isethionate ester surfactant of the formula (I) may be prepared by any of the methods disclosed in the prior art, for example see the methods described in WO 94/09763 and WO 2005/075623.

In some embodiments only a single isethionate ester surfactant of the formula (I) may be present in the cleansing composition of the first aspect. In some embodiments a mixture of two or more isethionate ester surfactants of the formula (I) may be present. In such embodiments the above amounts refer to the total amounts of all isethionate ester surfactants of the formula (I) present in the composition.

Preferably, M⁺ represents an optionally substituted ammonium cation or, most preferably, a metal cation. Suitable ammonium cations include NH₄⁺ and the ammonium cation of triethanolamine. Suitable metal cations include alkali metal cations, for example sodium, lithium and potassium cations, and alkaline earth metal cations, for example calcium and magnesium cations. Preferably M⁺ represents a zinc, potassium or sodium cation. Most preferably M⁺ represents a sodium cation.

The skilled person will appreciate that when M⁺ is a divalent metal cation two moles of anion will be present for each mole of cation.

Suitably, the cleansing composition of the first aspect comprises from 0.5 to 6 wt% of at least one alkyl isethionate ester surfactant of the formula (II), more suitably from 1 to 5 wt% of at least one alkyl isethionate ester surfactant of the formula (II), such as from 2 to 4 wt% of at least one alkyl isethionate ester surfactant of the formula (II), particularly from 2.5 to 3.5 wt% of at least one alkyl isethionate ester surfactant of the formula (II).

In the formula (II), the group R₂ represents a substituted or unsubstituted C₄-C₃₆ hydrocarbyl group, the groups R₃, R₄, R₅ and R₆ each independently represents hydrogen or a substituted or unsubstituted C₁-C₄ alkyl group, provided that at least one of R₃, R₄, R₅ and R₆ is not hydrogen, and M₁⁺ represents a cation.

Preferably, R₂ is selected from a substituted or unsubstituted C₄-C₃₆ alkyl, C₄-C₃₆ alkenyl, Cs-C₁₂ aryl or C₄-C₃₁ alkyl-C₅-C₁₂ aryl group. More preferably, R₂ is selected from a substituted or unsubstituted C₄-C₃₆ alkyl or C₄-C₃₆ alkenyl group. Most preferably, R₂ is an unsubstituted C₄-C₃₆ alkyl or C₄-C₃₆ alkenyl group, especially an unsubstituted C₄-C₃₆ alkyl group.

Preferably, R₂ represents a substituted or unsubstituted C₅-C₃₀ alkyl group, preferably a substituted or unsubstituted C₇-C₂₄ alkyl group, more preferably a substituted or unsubstituted C₇-C₂₁ alkyl group, most preferably a substituted or unsubstituted C₇-C₁₇ alkyl group.

Preferably, R₂ represents an unsubstituted C₅-C₃₀ alkyl group, preferably an unsubstituted C₇-C₂₄ alkyl group, more preferably an unsubstituted C₇-C₂₁ alkyl group, most preferably an unsubstituted C₇-C₁₇ alkyl group.

R₂ is preferably the residue of a fatty acid. Fatty acids obtained from natural oils often include mixtures of fatty acids. For example, the fatty acid obtained from coconut oil contains a mixture of fatty acids including C₁₂ lauric acid, C₁₄ myristic acid, C₁₆ palmitic acid, C₈ caprylic acid, and C₁₈ stearic and oleic acid.

R₂ may include the residue of one or more naturally occurring fatty acids and/or of one or more synthetic fatty acids. In some preferred embodiments, R₂ consists essentially of the residue of a single fatty acid.

Examples of carboxylic acids from which R₂ may be derived include coco acid, hexanoic acid, caproic acid, caprylic acid, capric acid, lauric acid, myristic acid, palmitic acid, palmitoleic acid, stearic acid, oleic acid, linoleic acid, arachidic acid, gadoleic acid, arachidonic acid, eicosapentanoic acid, behinic acid, eruic acid, docosahexanoic lignoceric acid, naturally occurring fatty acids such as those obtained from coconut oil, tallow, palm kernel oil, butterfat, palm oil, olive oil, corn oil, linseed oil, peanut oil, fish oil and rapeseed oil; synthetic fatty acids made as chains of a single length or a selected distribution of chain lengths; and mixtures thereof. Suitably R₂ comprises the residue of coco acid, the residue of mixed fatty acids derived from coconut oil or the residue of mixed fatty acids derived from palm kernel oil. More suitably. R₂ comprises the residue of a saturated fatty acid having 12 carbon atoms.

For example, the alkyl isethionate ester surfactants of the formula (II) may be selected from one or more of sodium lauroyl methyl isethionate, sodium cocoyl methyl isethionate and sodium oleoyl methyl isethionate. Sodium lauroyl methyl isethionate is especially preferred.

The cleansing composition of the first aspect may include a mixture of more than one compound of formula (II). For example an isomeric mixture of compounds of formula (II) may be present. Such a mixture may include, for example, a compound in which R₃ is substituted or unsubstituted C₁-C₄ alkyl (suitably methyl) and R₄, R₅ and R₆ are all hydrogen and a compound in which R₆ is substituted or unsubstituted C₁-C₄ alkyl (suitably methyl) and R₃, R₄ and R₅ are all hydrogen.

The alkyl isethionate ester surfactant of the formula (II) may be prepared by any of the methods disclosed in the prior art, for example see the methods described in WO 94/09763 and WO 2005/075623.

In some embodiments only a single alkyl isethionate ester surfactant of the formula (II) may be present in the cleansing composition of the first aspect, In some embodiments a mixture of two or more alkyl isethionate ester surfactants of the formula (II) may be present. In such embodiments the above amounts refer to the total amounts of all alkyl isethionate ester surfactants of the formula (II) present in the composition.

When any of R₃, R₄, R₅ and R₆ represents a substituted or unsubstituted C₁-C₄ alkyl group, the alkyl group is preferably n-propyl, ethyl or methyl, such as ethyl or methyl, most preferably methyl.

Preferably one of the groups R₃, R₄, R₅ and R₆ represents a substituted or unsubstituted C₁-C₄ alkyl group and the remaining groups represent hydrogen. For example, R₃ may represent a substituted or unsubstituted C₁-C₄ alkyl group and R₄, R₅ and R₆ may all represent hydrogen. For example, R₅ may represent a substituted or unsubstituted C₁-C₄ alkyl group and R₃, R₄ and Re may all represent hydrogen.

Preferably, R₃ represents an unsubstituted C₁-C₄ alkyl group and R₄, R₅ and R₆ all represent hydrogen. Preferably, R₅ represents an unsubstituted C₁-C₄ alkyl group and R₃. R₄ and Re all represent hydrogen.

Most preferably, R₃ represents a methyl group and R₄, R₅ and R₆ all represent hydrogen. Most preferably, R₅ represents a methyl group and R₃, R₄ and R₅ all represent hydrogen.

Preferably, M₁⁺ represents an optionally substituted ammonium cation or, most preferably, a metal cation. Suitable ammonium cations include NH₄⁺ and the ammonium cation of triethanolamine. Suitable metal cations include alkali metal cations, for example sodium, lithium and potassium cations, and alkaline earth metal cations, for example calcium and magnesium cations. Preferably, M₁⁺ represents a zinc, potassium or sodium cation. Most preferably, M₁⁺ represents a sodium cation.

The skilled person will appreciate that when M₁⁺ is a divalent metal cation two moles of anion will be present for each mole of cation.

When any of the groups R₁, R₂, R₃, R₄, R₅ and/or R₆ are substituted, suitable substituents may include non-hydrocarbon groups provided that they do not alter the predominantly hydrocarbon nature of the group R₁, R₂, R₃, R₄, R₅ and/or R₆. Examples of suitable substituents for any of the groups R₁, R₂, R₃, R₄, R₅ and/or R₆ include C₁₋₄ alkoxy, cyano, hydroxy, oxo, halo (especially fluoro and chloro), trifluoromethyl and trifluoromethoxy.

Suitably, the cleansing composition of the first aspect comprises from 55 to 95 wt% of soap, more suitably from 60 to 95 wt% of soap, such as from 70 to 95 wt% of soap, preferably from 70 to 85 wt% of soap.

Any suitable soap may be included in the cleansing composition of the first aspect. Suitable soaps include saponified oils and neutralised fatty acids. For example, the soap may be selected from one or more salts of C₈-C₂₂ saturated or unsaturated fatty acids, such as one or more of sodium cocoate, sodium tallowate, sodium laurate, sodium myristate, sodium stearate, sodium palmate, sodium palm kernalate, sodium olivate, potassium cocoate, potassium tallowate, potassium laurate, potassium myristate, potassium stearate, potassium palmate, potassium palm kernalate, potassium olivate, mono-, di- or tri-ethanolamine cocoate, mono-, di- or tri-ethanolamine tallowate, mono-, di- or tri-ethanolamine laurate, mono-, di- or tri-ethanolamine myristate, mono-, di- or tri-ethanolamine stearate, mono-, di- or tri-ethanolamine palmate, mono-, di- or tri-ethanolamine palm kernalate, and mono-, di- or tri-ethanolamine olivate.

Suitably, the soap may be selected from one or more of sodium cocoate, sodium tallowate, sodium laurate, sodium myristate, sodium stearate, sodium palmate, sodium palm kernalate and sodium olivate. More suitably, the soap may be selected from one or more of sodium cocoate and sodium tallowate. For example, the soap may comprise sodium cocoate and/or sodium tallowate, such as a mixture of sodium cocoate and sodium tallowate in a weight ratio in the range of from 1:4 to 4:1. Particularly, the soap may comprise a mixture of sodium cocoate and sodium tallowate in a weight ratio of 1:4.

The cleansing composition of the first aspect may comprise one or more further surfactants (such as one or more further solid surfactants) in addition to the surfactants of the formulae (I) and (II). Other suitable surfactants include anionic surfactants, cationic surfactants, non-ionic surfactants, zwitterionic and amphoteric surfactants.

Suitable anionic surfactants for use herein include sulfates, sulfonates, phosphates, salts of C₁₂ to C₁₈ carboxylic acids, ethoxylated carboxylic acids, ester carboxylates, ethoxylated ester carboxylates, sarcosinates, amino acid surfactants such as glutamates and glycinates, sulfosuccinates, taurates, lactylates and sulfoacetates.

Particularly exemplary salts of the above, where applicable, are the sodium, potassium, ammonium, magnesium and triethanolamine salts.

Particularly preferred anionic surfactants for use herein include sodium methyl cocoyl taurate, sodium lauryl sarcosinate, sodium lauryl sulfosuccinate, alkyl sulfates, for example sodium lauryl sulfate, and alkyl ether sulfates, for example sodium lauryl ether sulfate.

Suitable non-ionic surfactants for use herein include alcohol alkoxylates such as alcohol ethoxylates and alcohol propoxylates, ethylene oxide/propylene oxide copolymer derived surfactants, sugar esters, especially sorbitan esters, alkyl polyglucosides, fatty acid alkoxylates such as fatty acid ethoxylates and propoxylates, polyethylene glycol esters and partial esters, alkanolamides and amineoxides.

Suitable cationic surfactants for use herein are typically based on fatty amine derivates or phosphonium quaternary ions, and quaternary ammonium compounds.

Suitable amphoteric surfactants include those based on fatty nitrogen derivatives and those based on betaines.

Suitable amphoteric or zwitterionic surfactants may be selected from betaines, for example alkyl betaines, alkylamidopropyl betaines, alkylamidopropyl hydroxy sultaines, alkylampho acetates, alkylamphodiacetates, alkylamphopropionates, alkylamphodipropionates, alkyliminodipropionates and alkyliminodiacetate.

Suitably the cleansing composition of the first aspect may comprise from 0.1 to 10 wt%, suitably from 0.5 to 7.5 wt%, for example from 1 to 5 wt% or from 2 to 4 wt% of a further surfactant as discussed above.

The cleansing composition of the first aspect may compromise any suitable additional ingredients. Examples of suitable such additional ingredients include fragrances, pigments/colourants (such as titanium dioxide), fillers (such as talc or starch), waxes (such as paraffin, beeswax or Carnauba), polyquats (such as Polyquat 7, Polyquat 10, Polyquat 11 or Polyquat 22), poloxamers, polyhydroxy alcohols (such as glycerine, PEG), cationic guars, chelants (such as tetrasodium etidronate, ethylene-N,N'-disuccinic acid, pentasodium pentatate or tetrasodium ethylenediaminetetraacetic acid) and active ingredients (such as salicylic acid, benzoyl peroxide, sunscreen, botanical or antimicrobial agents).

The cleansing composition of the first aspect can be used to clean any suitable substrate, such as the skin or hair. In a second aspect, the present invention provides a method of cleansing the skin and/or hair comprising contacting the skin and/or hair with a cleansing composition according to the first aspect.

The method of the second aspect may suitably involve wetting the skin and/or hair and/or wetting the cleansing composition (for example solid cleansing composition) with water prior to contacting the cleansing composition with the skin and/or hair.

The method of the second aspect may include the step of rinsing the cleansing composition from the skin and/or hair with water.

Preferably, the method of the second aspect is a method of washing the skin and/or hair.

In a third aspect, the present invention provides a method of manufacturing a cleansing composition having reduced and/or eliminated grit formation, the method comprising admixing:
(i) from 1 to 15 wt% of at least one C₈-C₂₂ fatty acid;
(ii) from 2 to 25 wt% of at least one isethionate ester surfactant of the formula (I): 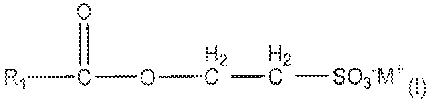
(iii) from 0.5 to 7.5 wt% of at least one alkyl isethionate ester surfactant of the formula (II) 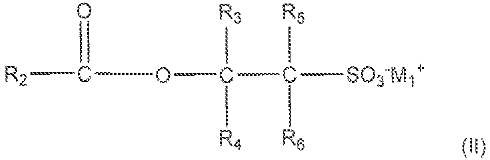
   wherein R₁ and R₂ each independently represents a substituted or unsubstituted C₄-C₃₆ hydrocarbyl group;
   R₃, R₄, R₅ and R₆ each independently represents hydrogen or a substituted or unsubstituted C₁-C₄ alkyl group, provided that at least one of R₃, R₄, R₅ and R₆ is not hydrogen; and
   M⁺ and M₁⁺ each independently represents a cation; and
(iv) from 52,5 to 95 wt% of soap.

The method of the third aspect may further comprise the step of compacting the cleansing composition to form the desired shape.

The method of the third aspect may comprise admixing the components in particulate form, pouring the mixed particulates into a mould and then compacting the mouid.

The method of the third aspect may comprise admixing and heating the components to a temperature (for example of 100°C or above) at which the components are melted or dispersed and then passing the resulting mixture through a chill roll and extruding the product into pellets. The pellets may then be mixed with further suitable additional ingredients before being refined and pressed into a bar.

The preferred features of the third aspect are as defined in relation to the first aspect.

In a fourth aspect, the present invention provides a base composition for use in a cleansing composition, the base composition comprising:
(i) from 10 to 40 wt% of at least one C₈-C₂₂ fatty acid; and
(ii) from 60 to 90 wt% of a synthetic detergent composition, wherein the synthetic detergent composition comprises from 65 to 95 wt% of at least one isethionate ester surfactant of the formula (I): 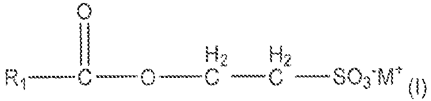 and from 5 to 35 wt% of at least one alkyl isethionate ester surfactant of the formula (II): 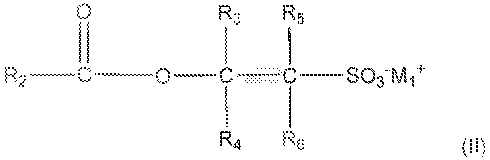
   wherein R₁ and R₂ each independently represents a substituted or unsubstituted C₄-C₃₆ hydrocarbyl group;
   R₃, R₄, R₅ and R₆ each independently represents hydrogen or a substituted or unsubstituted C₁-C₄ alkyl group, provided that at least one of R_{3,} R₄, R₅ and R₆ is not hydrogen; and
   M⁺ and M₁⁺ each independently represents a cation.

Suitably, the base composition of the fourth aspect is a solid composition. For example, the base composition may be in the form of a solid flake, noodle or chip. The base composition of the fourth aspect may be suitable for preparing a cleansing composition (such as a personal cleansing composition), especially a solid cleansing composition, for example as defined in relation to the first aspect.

The base composition of the fourth aspect may be used to provide a cleansing composition which offers the same advantages as the cleansing composition of the first aspect, as discussed herein. Additionally, the base composition of the fourth aspect may be supplied to manufacturers of cleansing compositions (especially solid cleansing compositions) for incorporation into such compositions using standard equipment and manufacturing processes.

The preferred features of the fourth aspect are as defined in relation to the first aspect. Thus, the preferred features of the C₈-C₂₂ fatty acid, isethionate ester surfactant of the formula (I) and alkyl isethionate ester surfactant of the formula (II) are as defined in relation to the first aspect.

The base composition of the fourth aspect may include a mixture of more than one compound of the formula (I) and/or (II), For example an isomeric mixture of compounds of formula (II) may be present. Such a mixture may include, for example a compound in which R₃ is substituted or unsubstituted C₁-C₄ alkyl (suitably methyl) and R₄, R₅ and R₆ are all hydrogen and a compound in which Re is substituted or unsubstituted C₁-C₄ alkyl (suitably methyl) and R₃, R₄ and R₅ are all hydrogen.

In some embodiments only a single C₈-C₂₂ fatty acid may be present in the base composition of the fourth aspect. In some embodiments a mixture of two or more C₈-C₂₂ fatty acids may be present. In such embodiments the above amounts refer to the total amounts of all C₈-C₂₂ fatty acids present in the composition.

In some embodiments only a single isethionate ester surfactant of the formula (I) may be present in the base composition of the fourth aspect. In some embodiments a mixture of two or more isethionate ester surfactants of the formula (I) may be present. In such embodiments the above amounts refer to the total amounts of all isethionate ester surfactants of the formula (I) present in the composition.

In some embodiments only a single alkyl isethionate ester surfactant of the formula (II) may be present in the base composition of the fourth aspect. In some embodiments a mixture of two or more alkyl isethionate ester surfactants of the formula (II) may be present. In such embodiments the above amounts refer to the total amounts of all alkyl isethionate ester surfactants of the formula (II) present in the composition.

In a fifth aspect, the present invention provides a cleansing composition (such as a personal cleansing composition) comprising a base composition according to the fourth aspect. Suitably, the present invention provides a cleansing composition (such as a personal cleansing composition) comprising a base composition according to the fourth aspect and soap.

Preferably, the present invention provides a solid cleansing composition comprising a base composition according to the fourth aspect. Preferably, the present invention provides a solid cleansing composition comprising a base composition according to the fourth aspect and soap.

A cleansing composition of the fifth aspect may comprise from 5 to 70 wt% of a base composition according to the fourth aspect and from 30 to 95 wt% of soap.

A cleansing composition of the fifth aspect may comprise from 5 to 47.5 wt% of a base composition according to the fourth aspect and from 52.5 to 95 wt% of soap.

A cleansing composition of the fifth aspect may comprise from 5 to 40 wt% of a base composition according to the fourth aspect and from 60 to 95 wt% of soap.

Any suitable soap may be included in the cleansing composition of the fifth aspect. Suitable soaps include saponified oils and neutralised fatty acids. For example, the soap may be selected from one or more salts of C₈-C₂₂ saturated or unsaturated fatty acids, such as one or more of sodium cocoate, sodium tallowate, sodium laurate, sodium myristate, sodium stearate, sodium palmate, sodium palm kernalate, sodium olivate, potassium cocoate, potassium tallowate, potassium laurate, potassium myristate, potassium stearate, potassium palmate, potassium palm kernalate, potassium olivate, mono-, di- or tri-ethanolamine cocoate, mono-, di- or tri-ethanolamine tallowate, mono-, di- or tri-ethanolamine laurate, mono-, di- or tri-ethanolamine myristate, mono-, di- or tri-ethanolamine stearate, mono-, di- or tri-ethanolamine palmate, mono-, di- or tri-ethanolamine palm kernalate, and mono-, di- or tri-ethanolamine olivate.

Suitably, the soap may be selected from one or more of sodium cocoate, sodium tallowate, sodium laurate, sodium myristate, sodium stearate, sodium palmate, sodium palm kernalate and sodium olivate. More suitably, the soap may be selected from one or more of sodium cocoate and sodium tallowate. For example, the soap may comprise sodium cocoate and/or sodium tallowate, such as a mixture of sodium cocoate and sodium tallowate in a weight ratio in the range of from 1:4 to 4:1. Particularly, the soap may comprise a mixture of sodium cocoate and sodium tallowate in a weight ratio of 1:4.

The cleansing composition of the fifth aspect may compromise any suitable additional ingredients. Examples of suitable such additional ingredients include fragrances, pigments/colourants (such as titanium dioxide), fillers (such as talc or starch), waxes (such as paraffin, beeswax or Carnauba), polyquats (such as Polyquat 7, Polyquat 10, Polyquat 11 or Polyquat 22). poloxamers, PEGs, cationic guars, chelants (such as tetrasodium etidronate, ethylene-N,N'-disuccinic acid, pentasodium pentatate or tetrasodium ethylenediaminetetraacetic acid) and active ingredients (such as salicylic acid, benzoyl peroxide, sunscreen, botanical or antimicrobial agents).

The cleansing composition of the fifth aspect can be used to clean any suitable substrate, such as the skin or hair. The present invention provides a method of cleansing the skin and/or hair comprising contacting the skin and/or hair with a cleansing composition according to the fifth aspect. This method includes the preferred features of the method of the second aspect.

The cleansing composition of the fifth aspect can be prepared by any suitable method, for example using a method according to the third aspect. The cleansing composition of the fifth aspect may be prepared by mixing a base composition according to the fourth aspect with soap.

In a sixth aspect, the present invention provides a method of reducing and/or eliminating grit formation in a cleansing composition (such as a personal cleansing composition, especially a solid cleansing composition), the method comprising incorporating a base composition according to the fourth aspect into the cleansing composition.

In a seventh aspect, the present invention provides a use of a base composition according to the fourth aspect to reduce and/or eliminate grit formation in a cleansing composition (such as a personal cleansing composition, especially a solid cleansing composition).

In an eighth aspect, the present invention provides a method of cleansing the skin and/or hair comprising contacting the skin and/or hair with a cleansing composition comprising a base composition according to the fourth aspect.

The preferred features of the eighth aspect are as defined in relation to the second aspect.

In a ninth aspect, the present invention provides a personal care product comprising a cleansing composition according to the first or fifth aspect and packaging.

Any suitable packaging may be used and will depend on the exact nature of the product.

### Examples

The invention will now be described with reference to the following non-limiting examples.

### Example 1

Solid cleansing compositions were prepared comprising the following ingredients, as shown in Table 1:

**Table 1**

| Composition | SCl / Fatty acid blend | SCl / Fatty acid / SLMI blend | Soap (%) | NaCl (wt%) | TiO₂ (wt%) |
|---|---|---|---|---|---|
| Example 1 | 0 | 24 | 73.3 | 2.5 | 0.2 |
| Comparative 2 | 24 | 0 | 73.3 | 2.5 | 0.2 |
| Example 3 | 0 | 34 | 63.3 | 2.5 | 0.2 |
| Comparative 4 | 34 | 0 | 63.3 | 2.5 | 0.2 |
| Example 5 | 0 | 14 | 83.3 | 2.5 | 0.2 |
| Comparative 6 | 14 | 0 | 83.3 | 2.5 | 0.2 |

| | | | | | |
|---|---|---|---|---|---|
| SCI = sodium cocoyl isethionate SLMI = sodium lauroyl methyl isethionate Soap = sodium cocoate and sodium tallowate in a weight ratio of 20:80 NaCl = 25% aqueous solution SCI / Fatty acid blend is about 65 wt% SCI and about 35 wt% C₈-C₂₂ fatty acid, predominantly stearic acid SCI / Fatty acid / SLMI blend is about 50 wt% SCI, about 15 wt% SLMI and about 35 wt% C₈-C₂₂ fatty acid, predominantly stearic acid | | | | | |

Flakes of SCI, fatty acid blend and SLMI (when present) and were dry mixed with the soap in an Amalgamator. Dry TiO₂ and the NaCl solution was then added and mixed. The resulting mixture was transferred to an extruder where it was refined with increasingly fine screens, 10 mesh, 30 mesh and 50 mesh. The material was then transferred to a plodder to make slugs that were pressed into soap bars. The resultant bars were tested for their grit content at 25°C and 30°C, their hardness and their processability. The results are provided in Table 2 below.

**Table 2**

| Composition | Grit at 25°C | Grit at 30°C | Hardness | Processability |
|---|---|---|---|---|
| Example 1 | 1 | 0 | 47 | Average |
| Comparative 2 | >7 | 5 | 48 | Average |
| Example 3 | 2 | 0 | 67 | Difficult |
| Comparative 4 | >7 | 6 | 65 | Difficult |
| Example 5 | 1 | 0 | 39 | Easy |
| Comparative 6 | >7 | 6 | 41 | Easy |

The processability was judged subjectively by the inventors. The factors considered included the softness of the pellets being extruded, the time it took to process, stickiness of the pellets as they are being processed and whether the material would pass through the hopper to the extruder.

The hardness was measured according to ASTM 1321.

For the grit test, each of the bars prepared was washed down in tap water at 25°C or 30°C. The bar was held in a stream of running tap water at the specified temperature and washed down for one minute by revolving it gently by rubbing it in the hands. At the end of this period, the surface texture of the wet bar was noted and the surface texture of the washed bar was then graded. The lower the grading then the lower the grit, with a grading of zero indicating that no grit was present. After the wash down test, the bars were allowed to dry for 24 hours, placed on a sheet of carbon black paper and held for 20 seconds. This enabled the presence of grit to be seen.

It can be seen from Table 2 that the bars according to the invention (namely compositions of Examples 1, 3 and 5) exhibit reduced or no grit when compared to the comparative examples. This is further shown in Figure 1, where the bars according to the invention are shown visually to be smoother and with less grit when compared to the comparative examples. Table 2 also shows that the bars according to the invention (namely compositions of Examples 1, 3 and 5) maintain the processability and hardness properties when compared to the comparative examples.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, and drawings), and/or all of the steps of any method or process so disclosed, may be combined in any combination, except combinations where at least some of such features and/or steps are mutually exclusive.

## Claims

1. A cleansing composition comprising:
(i) from 1 to 15 wt% of at least one C₈-C₂₂ fatty acid;
(ii) from 2 to 25 wt% of at least one isethionate ester surfactant of the formula (I): 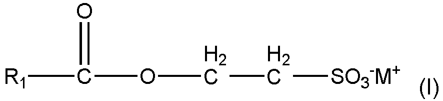
(iii) from 0.5 to 7.5 wt% of at least one alkyl isethionate ester surfactant of the formula (II): 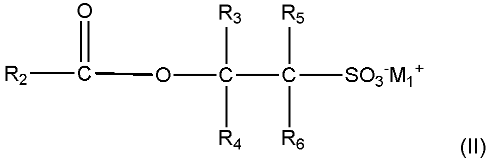
wherein R₁ and R₂ each independently represents a substituted or unsubstituted C₄-C₃₆ hydrocarbyl group;
R₃, R₄, R₅ and R₆ each independently represents hydrogen or a substituted or unsubstituted C₁-C₄ alkyl group, provided that at least one of R₃, R₄, R₅ and R₆ is not hydrogen; and
M⁺ and M₁⁺ each independently represents a cation; and
(iv) from 52.5 to 95 wt% of soap, preferably wherein the cleansing composition is a solid composition.

2. A cleansing composition according to claim 1, wherein the C₈-C₂₂ fatty acid is selected from one or more of stearic acid, coconut fatty acid, lauric acid, myristic acid and palmitic acid.

3. A cleansing composition according to claim 1 or 2, wherein the at least one isethionate ester surfactant of the formula (I) is selected from one or more of sodium lauroyl isethionate, sodium cocoyl isethionate and sodium myristoyl isethionate.

4. A cleansing composition according to any preceding claim, wherein the at least one alkyl isethionate ester surfactant of the formula (II) is selected from one or more of sodium lauroyl methyl isethionate, sodium cocoyl methyl isethionate and sodium oleoyl methyl isethionate.

5. A cleansing composition according to any preceding claim, wherein the soap is selected from one or more salts of C₈-C₂₂ saturated or unsaturated fatty acids, preferably wherein the soap is selected from one or more of sodium cocoate, sodium tallowate, sodium laurate, sodium myristate, sodium stearate, sodium palmate, sodium palm kernalate, sodium olivate, potassium cocoate, potassium tallowate, potassium laurate, potassium myristate, potassium stearate, potassium palmate, potassium palm kernalate, potassium olivate, mono-, di- or tri-ethanolamine cocoate, mono-, di- or tri-ethanolamine tallowate, mono-, di- or tri-ethanolamine laurate, mono-, di- or tri-ethanolamine myristate, mono-, di- or tri-ethanolamine stearate, mono-, di- or tri-ethanolamine palmate, mono-, di- or tri-ethanolamine palm kernalate, and mono-, di- or tri-ethanolamine olivate.

6. A cleansing composition according to any preceding claim, comprising one or more further surfactants selected from one or more of anionic surfactants, cationic surfactants, nonionic surfactants, zwitterionic and amphoteric surfactants.

7. A cleansing composition according to any preceding claim, comprising one or more additional ingredients selected from one or more of fragrances, pigments/colourants, fillers, waxes, polyquats, poloxamers, polyhydroxy alcohols, cationic guars, chelants and active ingredients.

8. A method of cleansing the skin and/or hair comprising contacting the skin and/or hair with a cleansing composition according to any preceding claim.

9. A method of manufacturing a cleansing composition having reduced and/or eliminated grit formation, the method comprising admixing:
(i) from 1 to 15 wt% of at least one C₈-C₂₂ fatty acid;
(ii) from 2 to 25 wt% of at least one isethionate ester surfactant of the formula (I): 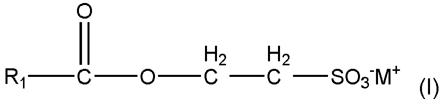
(iii) from 0.5 to 7.5 wt% of at least one alkyl isethionate ester surfactant of the formula (II): 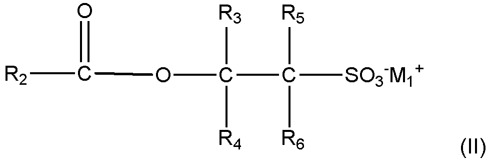
wherein R₁ and R₂ each independently represents a substituted or unsubstituted C₄-C₃₆ hydrocarbyl group;
R₃, R₄, R₅ and R₆ each independently represents hydrogen or a substituted or unsubstituted C₁-C₄ alkyl group, provided that at least one of R₃, R₄, R₅ and R₆ is not hydrogen; and
M⁺ and M₁⁺ each independently represents a cation; and
(iv) from 52.5 to 95 wt% of soap.

10. A base composition for use in a cleansing composition, the base composition comprising:
(i) from 10 to 40 wt% of at least one C₈-C₂₂ fatty acid; and
(ii) from 60 to 90 wt% of a synthetic detergent composition, wherein the synthetic detergent composition comprises from 65 to 95 wt% of at least one isethionate ester surfactant of the formula (I): 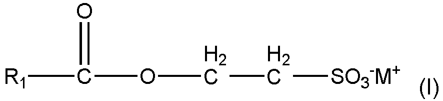 and from 5 to 35 wt% of at least one alkyl isethionate ester surfactant of the formula (II): 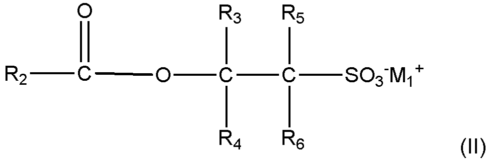
wherein R₁ and R₂ each independently represents a substituted or unsubstituted C₄-C₃₆ hydrocarbyl group;
R₃, R₄, R₅ and R₆ each independently represents hydrogen or a substituted or unsubstituted C₁-C₄ alkyl group, provided that at least one of R₃, R₄, R₅ and R₆ is not hydrogen; and
M⁺ and M₁⁺ each independently represents a cation, preferably wherein the base composition is a solid composition.

11. A cleansing composition comprising a base composition as defined in claim 10.

12. A method of reducing and/or eliminating grit formation in a cleansing composition, the method comprising incorporating a base composition according to claim 10 into the cleansing composition.

13. The use of a base composition according to claim 10 to reduce and/or eliminate grit formation in a cleansing composition.

14. A method of preparing a cleansing composition according to claim 11 comprising mixing a base composition according to claim 10 with soap.

15. A method of cleansing the skin and/or hair comprising contacting the skin and/or hair with a cleansing composition comprising a base composition according to claim 10.

16. A personal care product comprising a cleansing composition according to any of claims 1 to 7 and 11 and packaging.

## Patentansprüche

1. Reinigungszusammensetzung, umfassend:
(i) 1 bis 15 Gew.-% mindestens einer C₈-C₂₂-Fettsäure;
(ii) 2 bis 25 Gew.-% mindestens eines Isethionatester-Tensids der Formel (I): 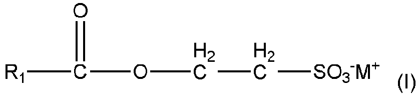
(iii) 0,5 bis 7,5 Gew.-% mindestens eines Alkylisethionatester-Tensids der Formel (II): 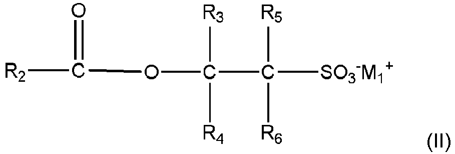
wobei R₁ und R₂ jeweils unabhängig für eine substituierte oder unsubstituierte C₄-C₃₆-Hydrocarbylgruppe stehen;
R₃, R₄, R₅ und R₆ jeweils unabhängig für ein Wasserstoffatom oder eine C₁-C₄-Alkylgruppe stehen, mit der Maßgabe, dass mindestens eines von R₃, R₄, R₅ und R₆ nicht für Wasserstoff steht; und
M⁺ und M₁⁺ jeweils für ein Kation stehen; und
(iv) 52,5 bis 95 Gew.-% Seife, vorzugsweise wobei es sich bei der Reinigungszusammensetzung um eine feste Zusammensetzung handelt.

2. Reinigungszusammensetzung nach Anspruch 1, wobei die C₈-C₂₂-Fettsäure aus Stearinsäure, Kokosnussfettsäure, Laurinsäure, Myristinsäure und Palmitinsäure ausgewählt ist.

3. Reinigungszusammensetzung nach Anspruch 1 oder 2, wobei das mindestens eine Isethionatester-Tensid aus Natriumlauroylisethionat, Natriumcocoylisethionat und/oder Natriummyristoylisethionat ausgewählt ist.

4. Reinigungszusammensetzung nach einem der vorhergehenden Ansprüche, wobei das mindestens eine Alkylisethionatester-Tensid der Formel (II) aus Natriumlauroylmethylisethionat, Natriumcocoylmethylisethionat und/oder Natriumoleoylmethylisethionat ausgewählt ist.

5. Reinigungszusammensetzung nach einem der vorhergehenden Ansprüche, wobei die Seife aus einem oder mehreren Salzen von gesättigten und ungesättigten C₈-C₂₂-Fettsäuren ausgewählt ist, vorzugsweise wobei die Seife aus Natriumcocoat, Natriumtallowat, Natriumlaurat, Natriummyristat, Natriumstearat, Natriumpalmat, Natriumpalmkernalat, Natriumolivat, Kaliumcocoat, Kaliumtallowat, Kaliumlaurat, Kaliummyristat, Kaliumstearat, Kaliumpalmat, Kaliumpalmkernalat, Kaliumolivat, Mono-, Di- oder Triethanolamincocoat, Mono-, Di- oder Triethanolamintallowat, Mono-, Di- oder Triethanolaminlaurat, Mono-, Di- oder Triethanolaminmyristat, Mono-, Di- oder Triethanolaminstearat, Mono-, Di- oder Triethanolaminpalmat, Mono-, Di- oder Triethanolaminpalmkernalat und Mono-, Di- oder Triethanolaminolivat ausgewählt ist.

6. Reinigungszusammensetzung nach einem der vorhergehenden Ansprüche, umfassend ein oder mehrere Tenside, die aus anionischen Tensiden, kationischen Tensiden, nichtionischen Tensiden, zwitterionischen und/oder amphoteren Tensiden ausgewählt sind.

7. Reinigungszusammensetzung nach einem der vorhergehenden Ansprüche, umfassend einen oder mehrere zusätzliche Inhaltsstoffe, die aus Duftstoffen, Pigmenten/Farbmitteln, Füllstoffen, Wachsen, Polyquats, Poloxameren, Polyhydroxyalkoholen, kationischen Guars, Chelatbildnern und/oder Wirkstoffen ausgewählt sind.

8. Verfahren zum Reinigen der Haut und/oder des Haars, bei dem man die Haut und/oder das Haar mit einer Reinigungszusammensetzung nach einem der vorhergehenden Ansprüche in Kontakt bringt.

9. Verfahren zur Herstellung einer Reinigungszusammensetzung mit verringerter und/oder eliminierter Kornbildung, bei dem man
(i) 1 bis 15 Gew.-% mindestens einer C₈-C₂₂-Fettsäure;
(ii) 2 bis 25 Gew.-% mindestens eines Isethionatester-Tensids der Formel (I): 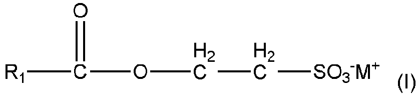
(iii) 0,5 bis 7,5 Gew.-% mindestens eines Alkylisethionatester-Tensids der Formel (II): 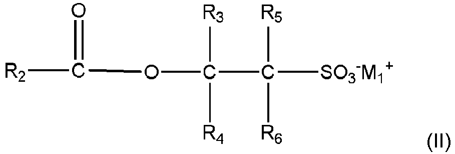
wobei R₁ und R₂ jeweils unabhängig für eine substituierte oder unsubstituierte C₄-C₃₆-Hydrocarbylgruppe stehen;
R₃, R₄, R₅ und R₆ jeweils unabhängig für ein Wasserstoffatom oder eine C₁-C₄-Alkylgruppe stehen, mit der Maßgabe, dass mindestens eines von R₃, R₄, R₅ und R₆ nicht für Wasserstoff steht; und
M⁺ und M₁⁺ jeweils für ein Kation stehen; und
(iv) 52,5 bis 95 Gew.-% Seife
mischt.

10. Basiszusammensetzung zur Verwendung in einer Reinigungszusammensetzung, wobei die Basiszusammensetzung Folgendes umfasst:
(i) 10 bis 40 Gew.-% mindestens einer C₈-C₂₂-Fettsäure und
(ii) 60 bis 90 Gew.-% einer synthetischen Detergenszusammensetzung, wobei die synthetische Detergenszusammensetzung 65 bis 95 Gew.-% mindestens eines Isethionatester-Tensids der Formel (I): 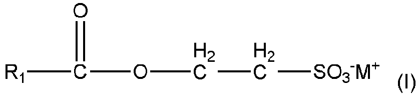 und 5 bis 35 Gew.-% mindestens eines Alkylisethionatester-Tensids der Formel (II): 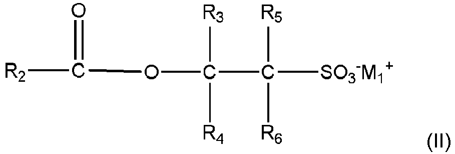
wobei R₁ und R₂ jeweils unabhängig für eine substituierte oder unsubstituierte C₄-C₃₆-Hydrocarbylgruppe stehen;
R₃, R₄, R₅ und R₆ jeweils unabhängig für ein Wasserstoffatom oder eine substituierte oder unsubstituierte C₁-C₄-Alkylgruppe stehen, mit der Maßgabe, dass mindestens eines von R₃, R₄, R₅ und R₆ nicht für Wasserstoff steht; und
M⁺ und M₁⁺ jeweils für ein Kation stehen;
umfasst, vorzugsweise wobei es sich bei der Basiszusammensetzung um eine feste Zusammensetzung handelt.

11. Reinigungszusammensetzung, umfassend eine Basiszusammensetzung gemäß Anspruch 10.

12. Verfahren zur Verringerung und/oder Eliminierung von Kornbildung in einer Reinigungszusammensetzung, bei dem man eine Basiszusammensetzung gemäß Anspruch 10 in die Reinigungszusammensetzung einarbeitet.

13. Verwendung einer Basiszusammensetzung nach Anspruch 10 zur Verringerung und/oder Eliminierung von Kornbildung in einer Reinigungszusammensetzung.

14. Verfahren zur Herstellung einer Reinigungszusammensetzung nach Anspruch 11, bei dem man eine Basiszusammensetzung nach Anspruch 10 mit Seife mischt.

15. Verfahren zum Reinigen der Haut und/oder des Haars, bei dem man die Haut und/oder das Haar mit einer Reinigungszusammensetzung, die eine Basiszusammensetzung nach Anspruch 10 umfasst, in Kontakt bringt.

16. Körperpflegeprodukt, umfassend eine Reinigungszusammensetzung nach einem der Ansprüche 1 bis 7 und 11 und eine Verpackung.

## Revendications

1. Composition de nettoyage comprenant :
(i) de 1 à 15 % en poids d'au moins un acide gras en C₈₋₂₂ ;
(ii) de 2 à 25 % en poids d'au moins un tensioactif de type ester d'iséthionate de formule (I) : 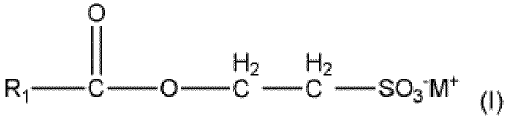
(iii) de 0,5 à 7,5 % en poids d'au moins un tensioactif de type ester d'alkyl-iséthionate de formule (II) : 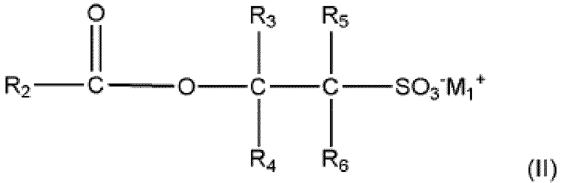
R₁ et R₂ représentant chacun indépendamment un groupe hydrocarbyle substitué ou non substitué en C₄₋₃₆ ;
R₃, R₄, R₅ et R₆ représentant chacun indépendamment hydrogène ou un groupe alkyle substitué ou non substitué en C₁₋₄, étant entendu qu'au moins un parmi R₃, R₄, R₅ et R₆ n'est pas hydrogène ; et
M⁺ et M₁⁺ représentant chacun indépendamment un cation ; et
(iv) de 52,5 à 95 % en poids de savon, préférablement la composition de nettoyage étant une composition solide.

2. Composition de nettoyage selon la revendication 1, l'acide gras en C₈₋₂₂ étant choisi parmi un ou plusieurs parmi l'acide stéarique, l'acide gras de noix de coco, l'acide laurique, l'acide myristique et l'acide palmitique.

3. Composition de nettoyage selon la revendication 1 ou 2, l'au moins un tensioactif de type ester d'iséthionate de formule (I) étant choisi parmi un ou plusieurs parmi le lauroyle iséthionate de sodium, le cocoyle iséthionate de sodium et le myristoyle iséthionate de sodium.

4. Composition de nettoyage selon une revendication précédente quelconque, l'au moins un tensioactif de type ester d'alkyl-iséthionate de formule (II) étant choisi parmi un ou plusieurs parmi le lauroyle méthyle iséthionate de sodium, le cocoyle méthyle iséthionate de sodium et l'oléoyle méthyle iséthionate de sodium.

5. Composition de nettoyage selon une revendication précédente quelconque, le savon étant choisi parmi un ou plusieurs sels d'acides gras saturés ou insaturés en C₈₋₂₂, préférablement le savon étant choisi parmi un ou plusieurs parmi le cocoate de sodium, le tallowate de sodium, le laurate de sodium, le myristate de sodium, le stéarate de sodium, le palmitate de sodium, le kernélate de palmier de sodium, l'olivate de sodium, le cocoate de potassium, le tallowate de potassium, le laurate de potassium, le myristate de potassium, le stéarate de potassium, le palmitate de potassium, le kernélate de palmier de potassium, l'olivate de potassium, le cocoate de monoéthanolamine, le cocoate de diéthanolamine et le cocoate de triéthanolamine, le tallowate de monoéthanolamine, le tallowate de diéthanolamine et le tallowate de triéthanolamine, le laurate de monoéthanolamine, le laurate de diéthanolamine et le laurate de triéthanolamine, le myristate de monoéthanolamine, le myristate de diéthanolamine et le myristate de triéthanolamine, le stéarate de monoéthanolamine, le stéarate de diéthanolamine et le stéarate de triéthanolamine, le palmitate de monoéthanolamine, le palmitate de diéthanolamine et le palmitate de triéthanolamine, le kernélate de palmier de monoéthanolamine, le kernélate de palmier de diéthanolamine et le kernélate de palmier de triéthanolamine, et l'olivate de monoéthanolamine, l'olivate de diéthanolamine et l'olivate de triéthanolamine.

6. Composition de nettoyage selon une revendication précédente quelconque, comprenant un ou plusieurs tensioactifs supplémentaires choisis parmi un ou plusieurs parmi des tensioactifs anioniques, des tensioactifs cationiques, des tensioactifs non ioniques, des tensioactifs zwitterioniques et amphotères.

7. Composition de nettoyage selon une revendication précédente quelconque, comprenant un ou plusieurs ingrédients supplémentaires choisis parmi un ou plusieurs parmi des parfums, des pigments/colorants, des charges, des cires, des polyquats, des poloxamères, des polyhydroxyalcools, des guars cationiques, des agents chélatants et des ingrédients actifs.

8. Procédé de nettoyage de la peau et/ou des cheveux comprenant la mise en contact de la peau et/ou des cheveux avec une composition de nettoyage selon une revendication précédente quelconque.

9. Procédé de fabrication d'une composition de nettoyage possédant une formation de grain réduite et/ou éliminée, le procédé comprenant le mélange :
(i) de 1 à 15 % en poids d'au moins un acide gras en C₈₋₂₂ ;
(ii) de 2 à 25 % en poids d'au moins un tensioactif de type ester d'iséthionate de formule (I) : 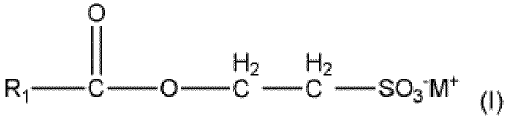
(iii) de 0,5 à 7,5 % en poids d'au moins un tensioactif de type ester d'alkyl-iséthionate de formule (II) : 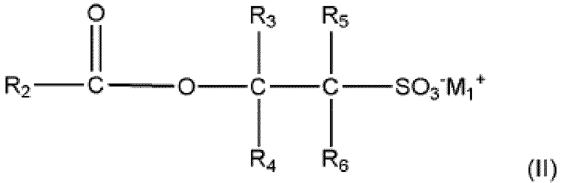
R₁ et R₂ représentant chacun indépendamment un groupe hydrocarbyle substitué ou non substitué en C₄₋₃₆ ;
R₃, R₄, R₅ et R₆ représentant chacun indépendamment hydrogène ou un groupe alkyle substitué ou non substitué en C₁₋₄, étant entendu qu'au moins un parmi R₃, R₄, R₅ et R₆ n'est pas hydrogène ; et
M⁺ et M₁⁺ représentant chacun indépendamment un cation ; et
(iv) de 52,5 à 95 % en poids de savon.

10. Composition de base pour une utilisation dans une composition de nettoyage, la composition de base comprenant
(i) de 10 à 40 % en poids d'au moins un acide gras en C₈₋₂₂ ; et
(ii) de 60 à 90 % en poids d'une composition de détergent synthétique, la composition de détergent synthétique comprenant de 65 à 95 % en poids d'au moins un tensioactif de type ester d'iséthionate de formule (I) : 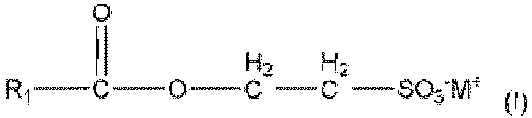 et de 5 à 35 % en poids d'au moins un tensioactif de type ester d'alkyl-iséthionate de formule (II) : 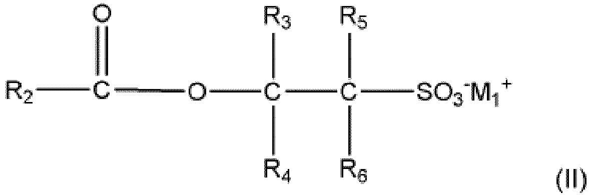
R₁ et R₂ représentant chacun indépendamment un groupe hydrocarbyle substitué ou non substitué en C₄₋₃₆ ;
R₃, R₄, R₅ et R₆ représentant chacun indépendamment hydrogène ou un groupe alkyle substitué ou non substitué en C₁₋₄, étant entendu qu'au moins un parmi R₃, R₄, R₅ et R₆ n'est pas hydrogène ; et
M⁺ et M₁⁺ représentant chacun indépendamment un cation, préférablement la composition de base étant une composition solide.

11. Composition de nettoyage comprenant une composition de base telle que définie dans la revendication 10.

12. Procédé de réduction et/ou d'élimination de formation de grain dans une composition de nettoyage, le procédé comprenant une incorporation d'une composition de base selon la revendication 10 dans la composition de nettoyage.

13. Utilisation d'une composition de base selon la revendication 10 pour réduire et/ou éliminer la formation de grain dans une composition de nettoyage.

14. Procédé de préparation d'une composition de nettoyage selon la revendication 11 comprenant le mélange d'une composition de base selon la revendication 10 avec du savon.

15. Procédé de nettoyage de la peau et/ou des cheveux comprenant la mise en contact de la peau et/ou des cheveux avec une composition de nettoyage comprenant une composition de base selon la revendication 10.

16. Produit de soin personnel comprenant une composition de nettoyage selon l'une quelconque des revendications 1 à 7 et 11 et un emballage.
